# EUROPEAN PATENT APPLICATION

(11) **EP 4 342 475 A1**
(43) Date of publication of application: **27.03.2024**
(21) Application number: 22804943.3
(22) Date of filing: 17.05.2022
(51) Int. Cl.: A61K 31/575, A61P 31/14, A61P 29/00

(54) **COMPOSITION FOR TREATMENT OF COVID-19 COMPRISING TAURODEOXYCHOLIC ACID OR PHARMACEUTICALLY ACCEPTABLE SALT THEREOF AS ACTIVE INGREDIENT**

(30) Priority: 20.05.2021 KR 20210064758
(71) Applicant: Shaperon Inc., Seoul 06373 (KR)
(72) Inventor: SEONG, Seung Yong, Seoul 05507 (KR); HAN, Seon Ae, Seoul 03357 (KR)
(74) Representative: Vossius & Partner Patentanwälte Rechtsanwälte mbB
(86) International application number: PCT/KR2022/007007
(87) International publication number: WO 2022/245089

(57) **Abstract**

The present invention relates to a composition for treatment of coronavirus disease 2019 (COVID-19) containing taurodeoxycholic acid (TDCA) or a pharmaceutically acceptable salt thereof as an active ingredient. Specifically, an early full recovery effect has been confirmed through the efficacy of treating clinical symptoms caused by novel severe acute respiratory syndrome coronavirus 2 (SARS-CoV-2) infection, alleviating inflammation, and inhibiting inflammatory cytokines using a pharmaceutically acceptable salt of taurodeoxycholic acid, and thus the taurodeoxycholic acid or a pharmaceutically acceptable salt thereof can be used as an active ingredient in a composition for prevention or treatment of lower respiratory tract infectious diseases, including coronavirus disease 2019.

## Description

### [Technical Field]

The present invention relates to a composition for treatment of coronavirus disease 2019 (COVID-19) containing taurodeoxycholic acid or a pharmaceutically acceptable salt thereof as an active ingredient, more particularly to a pharmaceutical composition for prevention or treatment of lower respiratory tract infectious diseases including coronavirus disease 2019 (COVID-19), which contains taurodeoxycholic acid or a pharmaceutically acceptable salt thereof as an active ingredient.

### [Background Art]

Coronavirus disease 2019 (COVID-19) is a pandemic that seriously threatens human health worldwide, with more than 200 million confirmed cases and more than 4 million deaths since its existence was first confirmed in 2019. COVID-19 is a severe acute respiratory syndrome disease spread by the novel coronavirus (SARS-CoV-2). SARS-CoV-2 belongs to the family Coronaviridae and is known to be a virus similar to SARS-CoV (Severe Acute Respiratory Syndrome Coronavirus) and MERS-CoV (Middle East Respiratory Syndrome Coronavirus). The host that is the clear source of infection and the infection route have not yet been confirmed, but it has been reported that infection is likely through contact with bats and that transmission through close contact between people is possible.

SARS-CoV-2 is a virus that can infect humans and various animals and is an RNA virus with a gene size of 27 to 32 kb. SARS-CoV-2 has an incubation period of about two weeks and mainly causes respiratory symptoms such as cough accompanied by fever, difficulty in breathing, shortness of breath, and phlegm, and in addition to these, may also cause headache, chills, runny nose, and muscle pain as well as digestive symptoms such as loss of appetite, nausea, vomiting, abdominal pain, and diarrhea.

Additionally, SARS-CoV-2 causes lower respiratory tract infections. Lower respiratory tract infections are diseases that develop when pathogens invade the respiratory tract located below the vocal cords, such as bronchitis, pneumonia, and pulmonary suppuration, and pneumonia is a representative lower respiratory tract infection. When pneumonia develops, the lungs become inflamed and the normal function of the lungs is impaired, resulting in pulmonary symptoms and systemic symptoms throughout the body. Pulmonary symptoms include coughing due to irritation of the respiratory system, phlegm due to discharge of inflammatory substances, and difficulty in breathing due to impaired breathing function, and digestive symptoms such as nausea, vomiting, and diarrhea may also occur. Additionally, systemic diseases throughout the body, such as headaches, fatigue, muscle pain, and joint pain, may develop.

SARS-CoV-2 may characteristically cause cytokine release syndrome or cytokine storm. Cytokine storm is a systemic inflammatory syndrome that may be caused by cancer, autoimmune disease, infectious disease, and the like and is characterized by excessive secretion of cytokines and hyperactivation of immune cells. This cytokine storm triggers a systemic immune response, resulting in high fever, lymph gland dysfunction, liver and spleen enlargement, cytopenia, hyperferritinosis, and central nervous system abnormalities, and may progress to multiple organ failure when left untreated. Cytokine storm also causes secondary dysfunction of various organs due to a hypersensitivity reaction of the systemic immune system, leading to patient death (Fajgenbaum et, al. N Engl J Med. 2020 Dec 3;383(23):2255- 2273). Currently, organ failure due to cytokine storm caused by COVID-19 has been identified as the leading cause of death, and pathologically, the main cause of cytokine storm is excessive activation of the NLRP3 inflammasome. Currently, corticosteroid-based steroids are generally concurrently administered to the patients to prevent cytokine storm, but there are limitations due to excessive inhibition of the systemic immune system and metabolic side effects.

To date, the representative drug officially approved by the US FDA for the treatment of hospitalized patients with COVID-19 is Remdesivir, an antiviral drug, and drugs to prevent systemic inflammation due to infection and resulting tissue damage are still limited. Currently, steroids such as dexamethasone are used as conservative management therapy to reduce the risk of death, and Tocilizumab, an IL-6 receptor blocker, and Baricitinib, a JAK inhibitor, have received emergency approval and are used as anti-inflammatory drugs, but various side effects are reported. According to the 'COVID-19 Treatment Guidelines Panel', the official COVID-19 guideline of the US NIH, experts from the FDA and NIH have mentioned the side effects and risks of steroid drugs, including dexamethasone, an adrenocortical steroid, and tocilizumab and baricitinib, and issued limitations and warnings that clinicians need to pay close attention. For example, long-term administration of steroids for more than 10 days is prohibited because of systemic metabolic side effects, and it is warned that it greatly increases the risk of secondary infections, such as opportunistic fungal infections or reactivation of viruses. In the case of tocilizumab, as a result of analyzing a total of 5 large-scale clinical trials, the evaluation of therapeutic efficacy often differed depending on the clinical trial, significant therapeutic effects have been confirmed only in two clinical trials, 'REMAP-CAP' and 'RECOVERY trials', and the limitation of low effect without concurrent use of steroids has been confirmed, so tocilizumab has received conditional emergency approval on the condition that tocilizumab must be used concurrently with steroids, and has been warned of possible side effects such as increased liver enzyme levels, neutropenia, thrombosis, and secondary infections. In the case of baricitinib, as a result of analyzing two large-scale clinical trials, the therapeutic effect has been confirmed in the 'ACTT-2 trial' but the therapeutic effect has been confirmed only in the critically ill patient group in the 'COV-BARRIER trial', which limits the interpretation of drug efficacy, so baricitinib has received conditional emergency approval on the condition that baricitinib must be used concurrently with steroids, and a warning has been issued against its use because of the observed risk of serious infection and blood clot-related side effects. Therefore, the development of therapeutic agents with fewer side effects while effectively reducing systemic inflammation caused by COVID-19 is continuously required. In addition, even if the nature of COVID-19 changes by the endemic, patients hospitalized by systemic inflammation are expected to continuously occur, and there is a possibility of a resurgence due to mutation, so anti-inflammatory drugs with guaranteed safety and effectiveness will be needed.

Excessive activation of the NLRP3 inflammasome due to activation of the P2X7 receptor after SARS-CoV-2 infection is the main cause of pneumonia in COVID-19 patients. The P2X7 receptors are distributed throughout the body, including immune cells, lung epithelial cells, and central nervous system cells, and when infection with pathogens, including SARS-CoV-2, occurs, (1) the interaction between the viral spike protein and the viral entry receptor ACE2 occurs, (2) angiotensin 2 rises, and (3) the ComC regulator is activated. In addition to the series of reactions, cell necrosis induced by virus proliferation and ATP released thereby activate the P2X7 receptor, causing excessive activation of the NLRP3 inflammasome, and the resulting systemic inflammatory response may lead the patient to death or severe sequelae such as pulmonary fibrosis.

The P2X7 receptor is an ion channel receptor composed of a complex belonging to the P2Xn purinergic receptor family, and extracellular release of potassium ions and rapid movement of calcium and sodium ions into cells are promoted when P2X7 is stimulated. In disease states such as infection, cells become necrotic, which greatly increases the concentration of extracellular ATP and activates the P2X7 receptor. Activated P2X7 activates the NLRP3 inflammasome, which is amplified by the release of potassium ions and intracellular ion imbalance. pro-IL-1β/18, in which NLRP3 inflammasome is activated, is activated and released into the tissue, intensifying the inflammatory response in surrounding tissues along with dead cells.

However, despite a detailed understanding of the activation mechanism of the NLRP3 inflammasome by P2X7, a P2X7 antagonist has not been developed. The variety of P2X7 subtypes in an organism and the variety of P2X7 polymorphisms between humans serve as major barriers to the development of P2X7 antagonists.

Bile acid is an important physiological molecule in bile secretion for absorption of lipids, harmful metabolites, and intestinal nutrients, and is produced in perivenous hepatocytes of the human liver. The primary bile acids formed in the human body are chenodeoxycholic acid and cholic acid. Chenodeoxycholic acid is conjugated with taurine or glycine to produce a total of eight conjugated bile acids, such as taurochenodeoxycholate and glycochenodeoxycholate. Additionally, by the influence of intestinal microorganisms on primary bile acids, secondary bile acids such as deoxycholic acid, lithocholic acid, and ursodeoxycholic acid are formed. Cholic acid is converted to deoxycholic acid and chenodeoxycholic acid is converted to lithocholic acid or ursodeoxycholic acid. Additionally, taurine is conjugated to deoxycholic acid to form taurodeoxycholic acid. These bile acids play an important role as signaling molecules in the absorption of dietary lipids, cholesterol homeostasis, and regulation of systemic endocrine functions, and are known to regulate the activity of the immune and metabolic systems by regulating various signal transduction pathways. For example, ursodeoxycholic acid, a secondary bile acid that exists only in a significantly small amount, has been proven to have a pharmacological effect of improving liver function and is applied as a therapeutic agent for liver disease. Although the anti-inflammatory effect of bile acid has been reported in some cases, reports on the detailed mechanism of action and particularly on the control of inflammasome activity are extremely limited.

Accordingly, the present inventors have made efforts to develop a new therapeutic agent that is safe and has fewer side effects while having excellent therapeutic effects in lower respiratory tract infectious diseases caused by coronaviruses such as SARS-CoV-2, and as a result, confirmed that taurodeoxycholic acid not only increases the expression of GPCR19 and decreases the expression of P2X7 by binding to GPCR19 but also increases the formation of the GPCR19-P2X7 complex, thereby inhibiting the activation of P2X7 in bone marrow-derived macrophages where inflammation is induced using a salt of taurodeoxycholic acid. GPCR19 exists as a complex bound to the P2X7 receptor and the cell membrane of keratinocytes, microglia, and macrophages, and in cells where inflammation is induced, the expression of GPCR19 significantly decreases and the expression of the P2X7 receptor significantly increases, amplifying the inflammatory response. The present inventors have confirmed that in these inflamed tissues, taurodeoxycholic acid binds to GPCR19 and inhibits the activation of P2X7, thereby inhibiting the activation of the NLRP3 inflammasome and blocking major inflammatory responses. In addition, the present inventors have confirmed that inflammation is alleviated in a sepsis animal model and clinical symptoms are alleviated and the treatment period is shortened in COVID-19 patients using a salt of taurodeoxycholic acid. Through this, the present inventors have revealed that taurodeoxycholic acid and a pharmaceutically acceptable salt thereof can be used as an active ingredient in a composition for prevention or treatment of lower respiratory tract infectious diseases, including COVID-19, and thus completed the present invention.

### [Summary of Invention]

### [Technical Problem]

To date, a specific antiviral drug having an excellent effect to treat COVID-19 has not been developed. Additionally, antibody therapeutic agents that neutralize the virus's ability to penetrate cells exhibit limited effectiveness only in mild patients. However, excessive activation of the NLRP3 inflammasome due to activation of the P2X7 receptor after SARS-CoV-2 infection is attracting attention as a main cause of pneumonia in moderate and severe COVID-19 patients, but it is difficult to develop P2X7 antagonists because of the variety of P2X7 polymorphisms.

In order to solve the above problem, the present invention intends to provide a fundamental treatment method to treat pneumonia caused by COVID-19 by identifying the hitherto unknown mechanism of interaction between GPCR19 and P2X7 and inhibiting the NLRP3 inflammasome by controlling the interaction between GPCR19 and P2X7.

Accordingly, an object of the present invention is to provide a composition for prevention or treatment of lower respiratory tract infectious diseases, containing taurodeoxycholic acid or a pharmaceutically acceptable salt thereof as an active ingredient.

Another object of the present invention is to provide a method for preventing or treating lower respiratory tract infectious diseases, which includes administering taurodeoxycholic acid or a pharmaceutically acceptable salt thereof to an individual.

Still another object of the present invention is to provide a use of taurodeoxycholic acid or a pharmaceutically acceptable salt thereof for preparing a pharmaceutical composition for prevention or treatment of lower respiratory tract infectious diseases.

### [Solution to Problem]

In order to achieve the objects of the present invention, the present invention provides a pharmaceutical composition for prevention or treatment of lower respiratory tract infectious diseases, containing taurodeoxycholic acid or a pharmaceutically acceptable salt thereof as an active ingredient.

The present invention also provides a pharmaceutical composition to be used for prevention or treatment of lower respiratory tract infectious diseases, which contains taurodeoxycholic acid or a pharmaceutically acceptable salt thereof as an active ingredient.

The present invention also provides a method for preventing or treating lower respiratory tract infectious diseases, which includes administering taurodeoxycholic acid or a pharmaceutically acceptable salt thereof to an individual.

In addition, the present invention provides a use of taurodeoxycholic acid or a pharmaceutically acceptable salt thereof for preparing a pharmaceutical composition for prevention or treatment of lower respiratory tract infectious diseases.

### [Advantageous Effects of Invention]

The present inventors have confirmed that a salt of taurodeoxycholic acid inhibits excessive inflammatory response by regulating the interaction between GPCR19 and P2X7 and thus inhibiting the activation of the NLRP3 inflammasome in bone marrow-derived macrophages where inflammation is induced. The present inventors have also confirmed that a salt of taurodeoxycholic acid alleviates inflammatory responses in a sepsis animal model, improves clinical symptoms occurring in patients with coronavirus disease 2019 (COVID-19) and pneumonia caused by COVID-19, and shortens the recovery period, and taurodeoxycholic acid or a pharmaceutically acceptable salt thereof can be used as an active ingredient in a composition for prevention or treatment of lower respiratory tract infectious diseases, including COVID-19.

### [Brief Description of Drawings]

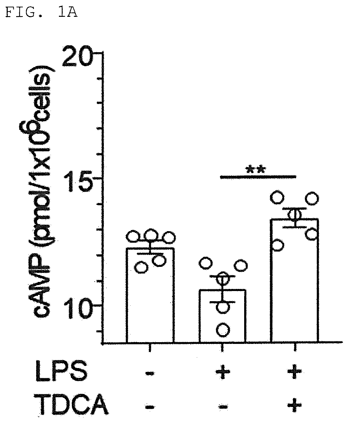

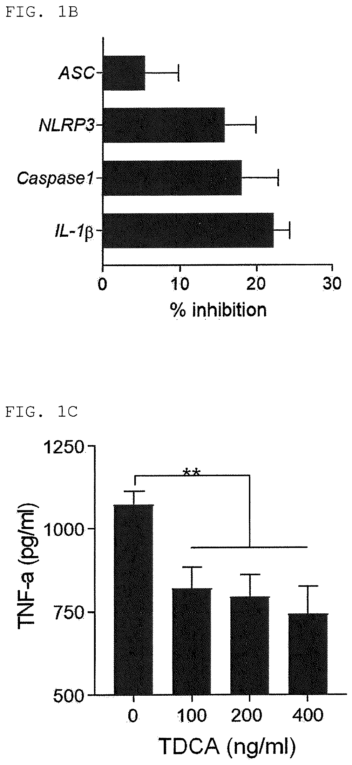

FIGS. 1A to 1C show that sodium taurodeoxycholate (hereinafter referred to as "TDCA") controls the priming step of inflammasome activation, and are diagrams illustrating changes in cAMP (FIG. 1A), inhibition of ASC, NLRP3, Caspase1, and IL-1β transcription (FIG. 1B), and inhibition of TNF-α production (FIG. 1C) by TDCA after induction of an inflammation priming signal through treatment with lipopolysaccharides (LPS) in an *in vitro* mouse bone marrow-derived macrophage (mBMDM) model.

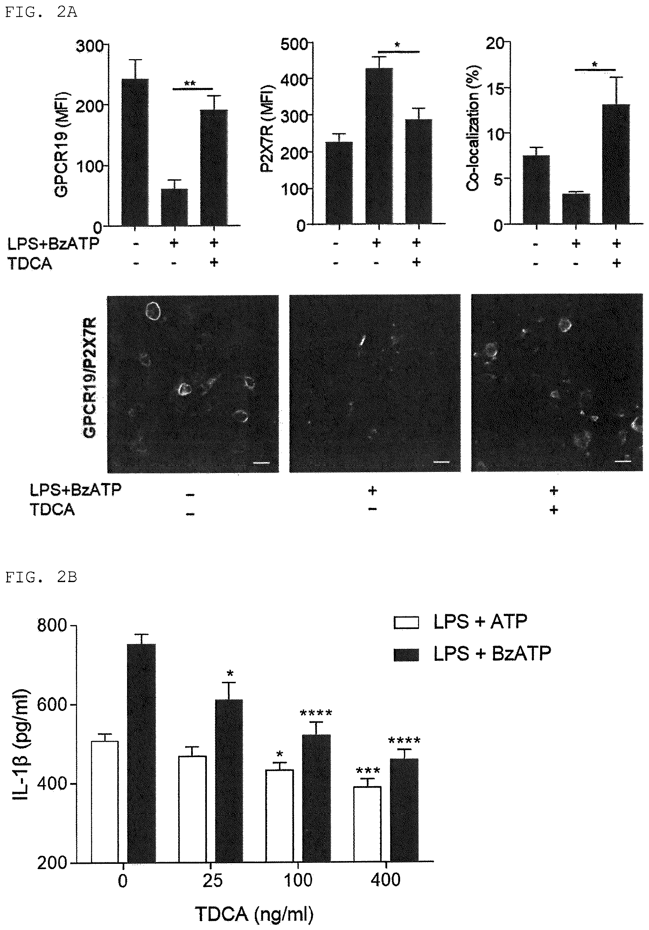

FIGS. 2A and 2B are diagrams showing that TDCA not only regulates the expression of GPCR19 and P2X7R, its receptors, but also regulates the physical binding between the two receptors, and are diagrams illustrating changes in GPCR19, P2X7R and their mutual binding by TDCA (FIG. 2A) after induction of an inflammation activation signal through treatment with LPS and ATP or BzATP in an *in vitro* mBMDM model and inhibition of IL-1β production by TDCA after treatment with BzATP, an agonist of P2X7R (FIG. 2B).

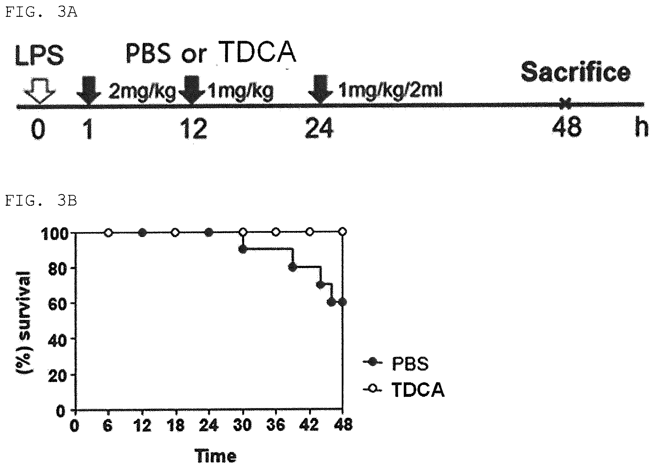

FIG. 3A is a diagram illustrating the method for constructing a sepsis model from a dog and administering TDCA.

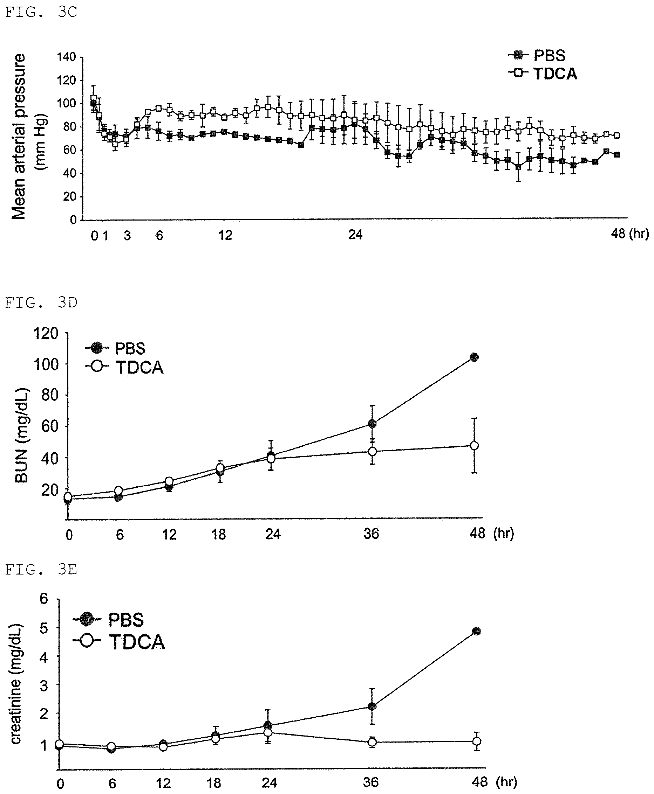

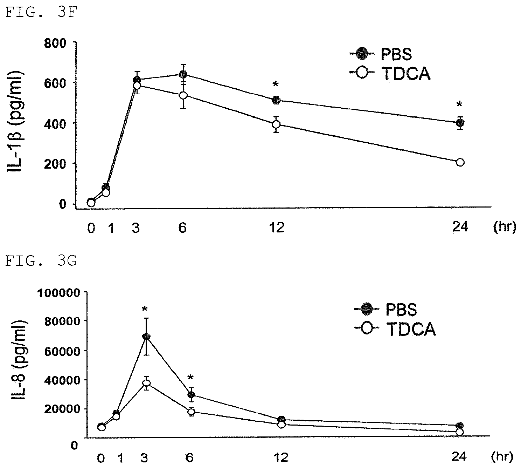

FIGS. 3B to 3G are diagrams illustrating the survival rate (FIG. 3B), blood pressure (FIG. 3C), blood urea nitrogen (BUN, FIG. 3D), creatinine (FIG. 3E), changes in IL-1β (FIG. 3F), and changes in IL-8 (FIG. 3G) according to TDCA administration in a canine sepsis model.

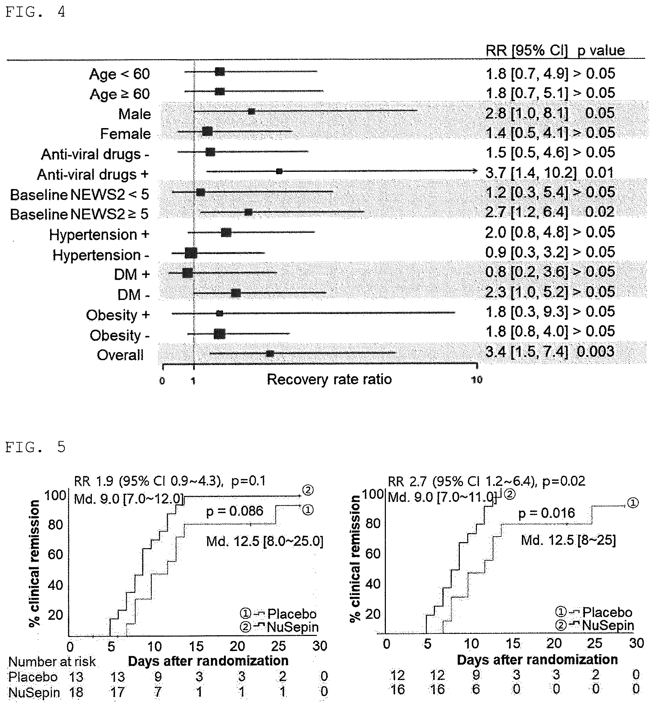

FIG. 4 is a diagram comparing the therapeutic efficiency of NuSepin 0.2mg/kg, an injection containing TDCA as an active ingredient, with placebo in patients with COVID-19 pneumonia. The therapeutic effect ratio of NuSepin 0.2 mg/kg compared to placebo has been analyzed using the Cox proportional hazard model by reflecting various variables affecting NEWS2 clinical evaluation, and at this time, NuSepin 0.2 mg/kg has a statistically significant therapeutic effect compared to placebo in an antiviral drug administration group and in moderate or severe patients at the time of hospital visit.
FIG. 5 is a diagram illustrating the effect of NuSepin 0.2 mg/kg intravenous injection in reducing duration of treatment compared to placebo in patients with COVID-19 pneumonia. In moderate to severe patients having NEWS2 of 5 or more on the clinical trial start date, the therapeutic efficiency determined by the NEWS2-based clinical evaluation index between the NuSepin 0.2 mg/kg administration group and the placebo group has been compared and analyzed using the Kaplan-Meier survival curve, and the recovery rate (RR) has been calculated using the Cox proportional hazard model. The left graph in FIG. 5 is a graph illustrating the proportion of patients who have recovered during the treatment period for the intention-to-treatment (ITT) analysis group, and the right graph in FIG. 5 is a graph illustrating the proportion of patients who have recovered during the treatment period for the per-protocol (PP) analysis group, which consists only of patients who have not violated the clinical protocol during a clinical trial:

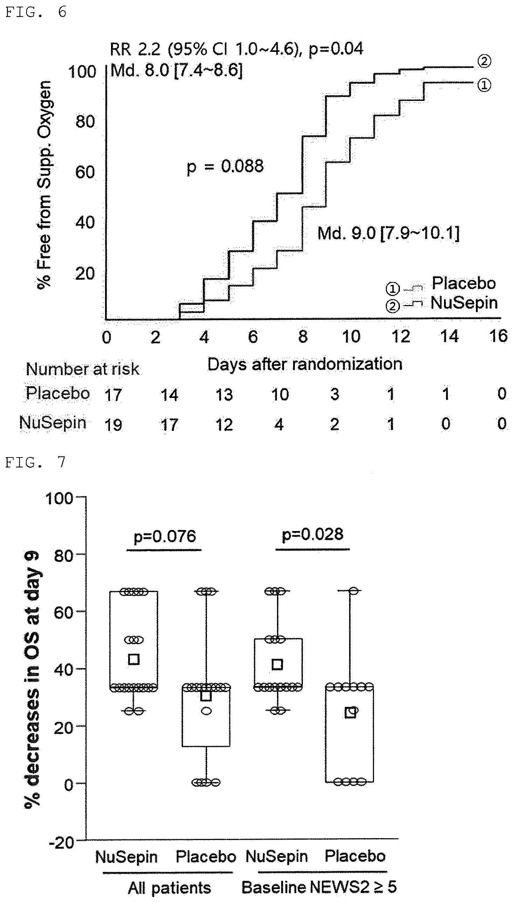

   FIG. 6 is a diagram illustrating the reduction in oxygen respirator dependence by intravenous injection of NuSepin 0.2 mg/kg or placebo in patients with COVID-19 pneumonia, and is a diagram analyzing the cumulative proportion of patients who does not require supplemental oxygen therapy between the NuSepin 0.2 mg/kg administration group and the placebo group using the Kaplan-Meier survival curve and Cox proportional hazard model in moderate and severe patients having NEWS2 > 5 or more at the time of hospitalization:
   FIG. 7 is a diagram illustrating the effect of NuSepin 0.2 mg/kg in improving the clinical status depending on the type of supplemental oxygen respirator in patients with COVID-19 pneumonia, and is a diagram comparing the rate of decrease of the ordinal scale, a clinical indicator, depending on the type of respirator compared to that on the start date (baseline) in order to compare the improvement in oxygen respirator dependence between patients administered with NuSepin 0.2 mg/kg and patients administered with placebo on day 9 of the clinical trial in the entire test patient group and in moderate to severe patients having a NEWS2 clinical score of 5 or more as of the clinical trial start date:

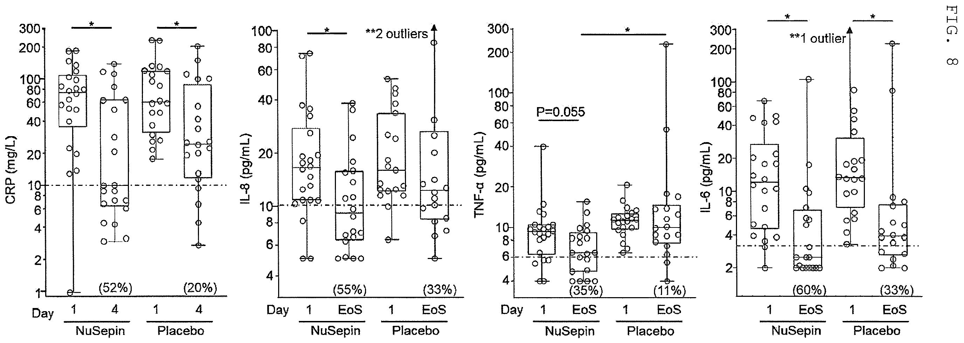

   FIG. 8 is a diagram illustrating the effect of NuSepin 0.2 mg/kg in reducing CRP and cytokines, which are inflammatory indicators in the blood, in patients with COVID-19 pneumonia, and is a diagram comparing blood CRP, IL-8, TNF-α, and IL-6 on the start date of clinical trial (day 1) and CRP on day 4 of clinical trial or blood cytokine concentration on the end date of clinical trial (EoS) in the NuSepin 0.2 mg/kg administration group and the placebo group in a COVID-19 pneumonia clinical trial. The proportion of patients having CRP or cytokine concentration in a normal range at the time of observation in the NuSepin 0.2 mg/kg group has been compared with that in the placebo group.

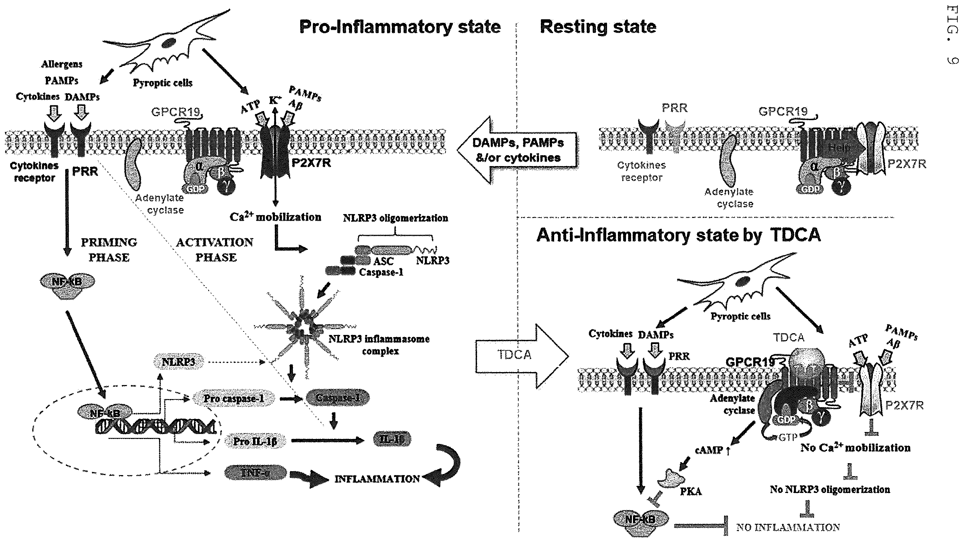

FIG. 9 is a diagram illustrating the mechanism by which TDCA inhibits excessive inflammatory response.

### [Description of Embodiments]

Hereinafter, the present invention will be described in more detail.

In the present invention, the term "prevention" refers to all actions to suppress or delay the occurrence, spread, and recurrence of lower respiratory tract infectious diseases by administration of the composition of the present invention, and the term "treatment" refers to all actions to improve or beneficially change the symptoms of the disease by administration of the composition of the present invention.

The present invention provides a pharmaceutical composition for prevention or treatment of lower respiratory tract infectious diseases, containing taurodeoxycholic acid or a pharmaceutically acceptable salt thereof as an active ingredient.

The present invention also provides a pharmaceutical composition to be used for prevention or treatment of lower respiratory tract infectious diseases, which contains taurodeoxycholic acid or a pharmaceutically acceptable salt thereof as an active ingredient.

The present invention also provides a method for preventing or treating lower respiratory tract infectious diseases, which includes administering taurodeoxycholic acid or a pharmaceutically acceptable salt thereof to an individual.

In addition, the present invention provides a use of taurodeoxycholic acid or a pharmaceutically acceptable salt thereof for preparing a pharmaceutical composition for prevention or treatment of lower respiratory tract infectious diseases.

In the present invention, as the taurodeoxycholic acid, taurodeoxycholic acid isolated from animal carcasses, for example, animals such as cattle, pigs, sheep, dogs, goats, or rabbits, commercially available taurodeoxycholic acid, or synthesized taurodeoxycholic acid can be all used freely.

The taurodeoxycholic acid is a type of bile acid, is in the form of taurine-conjugated deoxycholic acid, and has a chemical structure represented by the following [Chemical Formula 1], more specifically a chemical structure represented by the following [Chemical Formula 2] :

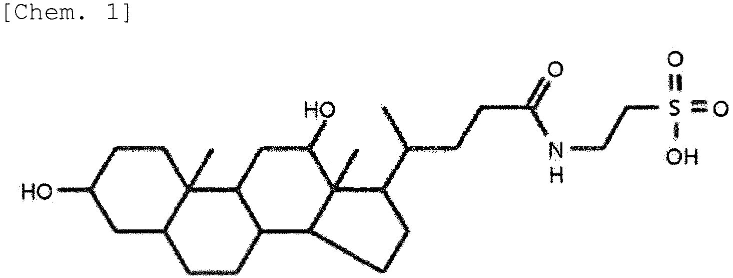

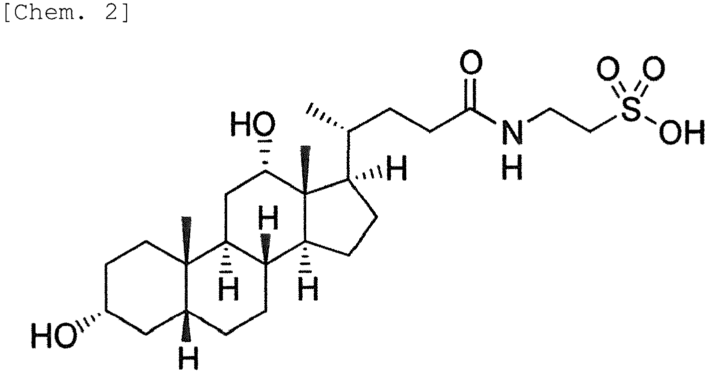

In the present invention, the taurodeoxycholic acid or a pharmaceutically acceptable salt thereof can inhibit the production of inflammatory cytokines, for example, TNF-α, IL-1β, IL-8, or IL-6.

The taurodeoxycholic acid or a pharmaceutically acceptable salt thereof is a GPCR19 agonist and can inhibit the activation of NF-κB by increasing cAMP and inhibit the activation of the NLRP3 inflammasome by inhibiting the function of P2X7, thereby inhibiting production of the inflammatory cytokines as illustrated in the schematic diagram of FIG. 9.

In the present invention, the lower respiratory tract infectious disease may be a disease having one or more symptoms selected from the group consisting of fever, cough, phlegm, body aches, sore throat, diarrhea, conjunctivitis, headache, skin rash, difficulty in breathing, acute bronchitis, pneumonia, pulmonary suppuration, renal failure, renal dysfunction, septic shock, sepsis, and cytokine release syndrome caused by coronavirus infection, but is not limited thereto.

The coronavirus may be one or more selected from the group consisting of Middle East respiratory syndrome coronavirus (MERS-CoV), severe acute respiratory syndrome coronavirus (SARS-CoV), and novel severe acute respiratory syndrome coronavirus 2 (SARS-CoV-2), specifically may be novel severe acute respiratory syndrome coronavirus 2 (SARS-CoV-2), but is not limited thereto.

The present invention includes not only taurodeoxycholic acid but also its pharmaceutically acceptable salts and possible solvates, hydrates, racemates, or stereoisomers that can be prepared therefrom.

Taurodeoxycholic acid of the present invention can be used in the form of a pharmaceutically acceptable salt, and an acid addition salt formed from a pharmaceutically acceptable free acid is useful as the salt. Acid addition salts are obtained from inorganic acids such as hydrochloric acid, nitric acid, phosphoric acid, sulfuric acid, hydrobromic acid, hydroiodic acid, nitrous acid or phosphorous acid, and non-toxic organic acids such as aliphatic mono- and di-carboxylates, phenyl-substituted alkanoates, hydroxyalkanoates and alkanedioates, aromatic acids, and aliphatic and aromatic sulfonic acids. These pharmaceutically non-toxic salts include a sulfate, pyrosulfate, bisulfate, sulfite, bisulfite, nitrate, phosphate, monohydrogen phosphate, dihydrogen phosphate, metaphosphate, pyrophosphate chloride, bromide, iodide, fluoride, acetate, propionate, decanoate, caprylate, acrylate, formate, isobutyrate, caprate, heptanoate, propiolate, oxalate, malonate, succinate, suberate, sebacate, fumarate, maleate, butyne-1,4-dioate, hexane-1,6-dioate, benzoate, chlorobenzoate, methyl benzoate, dinitro benzoate, hydroxybenzoate, methoxybenzoate, phthalate, terephthalate, benzenesulfonate, toluenesulfonate, chlorobenzenesulfonate, xylenesulfonate, phenylacetate, phenylpropionate, phenylbutyrate, citrate, lactate, hydroxybutyrate, glycolate, malate, tartrate, methanesulfonate, propanesulfonate, naphthalene-1-sulfonate, naphthalene-2-sulfonate, or mandelate.

The acid addition salt according to the present invention can be prepared by conventional methods, for example, by dissolving taurodeoxycholic acid in an excess of aqueous acid solution and precipitating the salt using a water-miscible organic solvent, such as methanol, ethanol, acetone, or acetonitrile. The acid addition salt can also be prepared by evaporating the solvent or excess acid from this mixture for drying, or suction filtering the precipitated salt.

A pharmaceutically acceptable metal salt can be prepared using a base. An alkali metal or alkaline earth metal salt is obtained, for example, by dissolving the compound in an excess of alkali metal hydroxide or alkaline earth metal hydroxide solution, filtering the undissolved compound salt, and evaporating the filtrate for drying. At this time, it is pharmaceutically appropriate to prepare a sodium, potassium, or calcium salt as the metal salt, and more specifically, it is appropriate to prepare a sodium salt. The corresponding silver salt is obtained by reacting the alkali metal or alkaline earth metal salt with a suitable silver salt (for example, silver nitrate).

When the composition is formed into a preparation, the preparation is prepared using diluents or excipients such as commonly used fillers, extenders, binders, wetting agents, disintegrants, and surfactants.

Solid preparations for oral administration include tablets, pills, powders, granules, capsules, troches, and the like, and these solid preparations are prepared by mixing taurodeoxycholic acid or a pharmaceutically acceptable salt thereof of the present invention with at least one or more excipients, such as starch, calcium carbonate, sucrose or lactose, or gelatin. In addition to simple excipients, lubricants such as magnesium stearate, and talc are also used. Liquid preparations for oral administration include suspensions, oral solutions, emulsions, or syrups, and may contain various excipients such as wetting agents, sweeteners, fragrances, and preservatives in addition to commonly used simple diluents such as water and liquid paraffin.

Preparations for parenteral administration include sterilized aqueous solutions, non-aqueous solutions, suspensions, emulsions, freeze-dried preparations, suppositories, and the like.

As non-aqueous solvents and suspending agents, propylene glycol, polyethylene glycol, vegetable oil such as olive oil, and injectable esters such as ethyl oleate may be used. As base materials for suppositories, witepsol, macrogol, tween 61, cacao oil, laurin oil, glycerol, gelatin, and the like may be used.

The composition according to the present invention is administered in a pharmaceutically effective amount. In the present invention, the "pharmaceutically effective amount" means an amount sufficient to treat a disease with a reasonable benefit/risk ratio applicable to medical treatment, and the effective dose level may be determined based on factors including the type and severity of the patient's disease, activity of the drug, sensitivity to the drug, time of administration, route of administration and excretion rate, treatment period, and drugs used concurrently, and other factors well known in the medical field. The composition of the present invention may be administered as an individual therapeutic agent or concurrently with other therapeutic agents, may be administered sequentially or simultaneously with conventional therapeutic agents, and may be administered singly or multiple times. It is important to administer the composition of the present invention in an amount so that the maximum effect can be acquired with the minimum amount without side effects, taking into account all of the above factors, and this may be easily determined by those skilled in the art.

Specifically, the effective amount of the compound according to the present invention may vary depending on the patient's age, gender, and body weight, and may generally be administered in an amount of 0.01 mg to 100 mg, more specifically 0.1 mg to 15 mg per kg of body weight, every day or every other day, or 1 to 3 times per day. However, since the effective amount may be increased or decreased depending on the route of administration, severity, gender, body weight, age, and the like, the dosage does not limit the scope of the present invention in any way.

### [Examples]

Hereinafter, the present invention will be described in detail with reference to Examples and Experimental Examples.

However, the following Examples and Experimental Examples only illustrate the present invention, and the contents of the present invention are not limited to the following Examples.

### <Example 1> Construction of bone marrow-derived macrophages and analysis of inflammatory cytokines through inflammasome stimulation

### <1-1> Construction of bone marrow-derived macrophages

Bone marrow-derived macrophages were constructed as follows.

Specifically, the bone marrow extracted from C57BL/6N mouse was differentiated in RPMI medium supplemented with 10% FBS, 100 U/ml penicillin streptomycin, 55 µM 2-mercaptoethanol, and 10 ng/ml recombinant mouse granulocyte-macrophage colony-stimulating factor (GM-CSF) for 7 days. On day 7, the proportion of differentiated macrophages was measured using flow cytometry and, as a result, was 94% to 96%.

### <1-2> Analysis of ability to inhibit production of inflammatory cytokines IL-1β and TNF-α derived from inflammation priming signals

The bone marrow-derived macrophages of Example <1-1> were cultured in culture dishes at various densities and then collected (4 × 10⁴, 5 × 10⁵, 1 × 10⁶ cells/well, 96-well, 24-well, and 12-well culture dishes, respectively). The next day, the bone marrow-derived macrophages were treated with 300 µM BzATP together with 10 ng/ml LPS derived from *Salmonella enterica serotype enteritidis,* or 300 µM BzATP and 400 ng/ml sodium taurodeoxycholate (hereinafter referred to as 'TDCA') for 1 hour in order to evaluate NLRP3 inflammasome components ASC, NLRP3, and Caspase1 as factors related to inflammation priming signals and an inflammatory cytokine IL-1β derived from the inflammation priming signals. After the time expired, the cells were collected, stored at -80°C, and then subjected to qRT-PCR analysis.

In order to evaluate an inflammatory cytokine TNF-α, the bone marrow-derived macrophages were treated with 10 ng/ml LPS for 3 hours to activate inflammation priming signals, then TDCA was added at various concentrations (0, 25, 100, 400 ng/ml), and the macrophages were cultured for 1 hour. After the time expired, the supernatant was collected, stored at -80°C, and then subjected to ELISA analysis.

### <1-3> Analysis of ability to inhibit production of inflammatory cytokine IL-1β derived from inflammation activation signals

The bone marrow-derived macrophages of Example <1-1> were cultured in culture dishes at various densities and then collected (4 × 10⁴, 5 × 10⁵, 1 × 10⁶ cells/well, 96-well, 24-well, and 12-well culture dishes, respectively). The next day, the bone marrow-derived macrophages were treated with 10 ng/ml LPS and 300 µM BzATP for 3 hours and then treated with TDCA at different specified concentrations (0, 25, 100, 400 ng/ml) for 1 hour in order to evaluate TGR5 and P2X7 as factors related to inflammation activation signals and an inflammatory cytokine IL-1β derived from the signals. After the time expired, the supernatant was collected, stored at -80°C, and then subjected to ELISA analysis.

### <Example 2> Analysis of inflammatory cytokines in sepsis animal model

### <2-1> Construction of sepsis animal model

To two treatment groups consisting of Mongrel dogs weighing 30 to 35 kg for each experimental group, 0.5 mg/kg/ml of LPS was administered intravenously according to the protocol illustrated in FIG. 3A to induce sepsis, then 2 mg/kg/2 ml of TDCA was injected intravenously (PBS was administered to the placebo group) 1 hour later, and 1 mg/kg/2 ml of TDCA was administered intravenously 12 and 24 hours later. After LPS administration, blood pressure was monitored every 1 hour, blood was collected at 0, 1, 3, 6, 12, 18, 24, 36, and 48 h, and the animals were sacrificed at 48 h to complete the experiment.

### <2-2> Survival rate analysis

The survival rate was examined by checking the number of beagle dogs that died for 48 hours in the two treatment groups in which sepsis was induced in Example <2-1>.

### <2-3> Blood pressure and renal function analysis

Blood pressure was measured hourly for 48 hours in the two treatment groups in which sepsis was induced in Example <2-1>.

Blood urea nitrogen (BUN) and creatinine tests were performed as renal function tests. Specifically, blood was collected hourly for 48 hours from the two treatment groups in which sepsis was induced in Example <2-1>, serum was separated, and then BUN and creatine concentrations in the serum were measured.

### <2-4> Analysis of inflammatory cytokines IL-1β and IL-8

Blood was collected hourly for 48 hours from the two treatment groups in which sepsis was induced in Example <2-1>, serum was separated, and then the inflammatory cytokine IL-1β in the serum was measured.

### <Example 3> Examination of safety of TDCA in human body - Phase 1 clinical trial

### <3-1> Selection of clinical trial subjects

Forty healthy adult male volunteers in Korea were selected as subjects for the clinical trial. The clinical trial was conducted at Seoul National University Hospital (Seoul, Korea) and was targeted at people who met all of the selection criteria in [Table 1].

**[Table 1]**

| Order | Selection criteria |
|---|---|
| 1 | Healthy male over 19 years of age but under 45 years of age at the time of screening visit |
| 2 | Those having BMI of 18.0 kg/m² or more and 27.0 kg/m² or less as a result of measurement at the time of screening visit |
| 3 | Those who are confirmed to be clinically healthy as a result of medical history survey, physical examination, vital signs, electrocardiogram (ECG) test, and appropriate clinical laboratory tests (however, even those outside the normal range can participate at the researcher's discretion) |
| 4 | Those who voluntarily decide to participate in this clinical trial and agree in writing to comply with the clinical trial compliance requirements |

However, subjects who met the items shown in [Table 2] below were excluded.

**[Table 2]**

| Order | Exclusion criteria |
|---|---|
| 1 | Those having a history of hypersensitivity reaction to drugs containing a taurodeoxycholate component or general drugs (aspirin, antibiotics, and the like) or clinically significant hypersensitivity reaction |
| 2 | Those who have shown clinical symptoms suspected of being an acute infectious disease within 2 weeks before the first administration date or whose body temperature (eardrum) measured at the time of screening is 38.0°C or more |
| 3 | Those who have or have a history of clinically significant diseases related to the liver, kidney, digestive system, respiratory system, musculoskeletal system, endocrine system, neuropsychiatric system, blood/tumor system, cardiovascular system and the like |
| 4 | Those having a history of gastrointestinal disease (for example: Crohn's disease, ulcer) or surgery (however, excluding simple appendectomy or hernia surgery) that may affect the absorption of the investigational drug |
| 5 | Those who have a history of drug abuse or who test positive for drugs of concern for abuse in a urine drug screening test |
| 6 | Those having the following blood pressure measured in a sitting position after resting for 3 minutes or more at the time of screening visit: |
| | A. Systolic blood pressure (SBP): < 90 mmHg or > 150 mmHg |
| | B. Diastolic blood pressure (DBP): < 50 mmHg or > 90 mmHg |
| 7 | Those who have participated in another clinical trial or bioequivalence study and administered with a drug within 6 months before the first administration date (the period is determined based on the last administration date of the previous trial). |
| 8 | Those who have donated whole blood within 2 months or apheresis blood within 1 month or received a blood transfusion within 1 month before the first administration date |
| 9 | Those who have taken drugs that induce or inhibit drug-metabolizing enzymes, such as barbiturates, within 1 month before screening |
| 10 | Those who have consumed food containing grapefruit/caffeine within 3 days of the first administration and those who cannot refrain from consuming food containing grapefruit from 3 days before hospitalization until the day of discharge |
| 11 | Those who have taken a prescription drug or herbal medicine within 2 weeks or an over-the-counter (OTC) drug within 1 week before the first administration date (however, at the investigator's discretion, they may participate in the clinical trial if other conditions are appropriate) |
| 12 | Those who consume too much caffeine, drink too much alcohol, or smoke too much (caffeine > 5 units/day, alcohol > 21 units/week (1 unit = 10 mL of pure alcohol), cigarettes > 10 cigarettes/day) |
| 13 | Those who have unusual eating habits (for example: consistent vegetarian eating habits) or who are unable to consume the meals provided in this clinical trial |
| 14 | Those determined by the investigator to be unsuitable as clinical trial subjects |

During the clinical trial, subjects who met the criteria in [Table 3] dropped out.

**[Table 3]**

| Order | Dropout criteria |
|---|---|
| 1 | A case where a test subject requests discontinuation of administration of the investigational drug during the clinical trial or withdraws consent to participate in the trial |
| 2 | A case where a test subject is administered with a drug that is expected to affect the evaluation of the safety or pharmacokinetics of the study drug during the clinical trial |
| 3 | A case where a serious adverse event/adverse drug event occurs |
| 4 | A case where it is impossible to collect blood for pharmacokinetics/safety test from a test subject |
| 5 | A case where a serious protocol violation is newly discovered during the clinical trial |
| 6 | A case were there is a request for discontinuation from the sponsor or the Ministry of Food and Drug Safety |
| 7 | Other cases where the clinical trial director/person in charge judges that the trial should be stopped |

### <3-2> Progress and management of phase 1 clinical trial

A randomized, double-blind, standard treatment-based placebo-controlled trial group was assigned to evaluate drug safety/tolerability and pharmacokinetics (PK) in clinical trial subjects. As a therapeutic agent, NuSepin, an injectable clinical agent containing TDCA, was used.

### [Testing method]

A screening test was performed on healthy male volunteers within 4 weeks (-28d to -4d) from the first administration date (1d) of the study drug to select test subjects determined to be suitable for this clinical trial. The selected test subjects were admitted to the Clinical Trial Center at Seoul National University Hospital in the afternoon 4 days before administration of the study drug, and randomly assigned to either the NuSepin 0.1, 0.2, 0.4, 0.8, or 1.6 mg/kg dose administration group or the placebo group, and then pharmacokinetic blood collection was performed for 3 days (-3d to -1d) to check the TDCA baseline. In the morning on the study drug administration date (1d), designated tests such as blood collection for pharmacokinetic analysis and safety evaluation were performed, and then the study drug or placebo was administered intravenously on an empty stomach. The test subjects were subjected to the clinical trial according to the set schedule and discharged from the hospital 1 day (2d) after administration, and the subjects who completed all clinical trial schedules underwent a post-study visit between 4d and 6d.

### [Evaluation method]

### • Evaluation of safety and tolerability

- Monitoring of adverse events such as subjective and objective symptoms
- Physical examination
- Vital signs
- Electrocardiogram (12-Lead ECG) test
- Clinical laboratory test
- Local toxicity assessment
- Continuous ECG and SpO2 monitoring

### • Pharmacokinetic evaluation

Blood was collected for pharmacokinetic evaluation of HY209 up to 24 hours after intravenous administration of the study drug;
- Blood collection time: 0 hours, 1 hour, 2 hours, 4 hours, 8 hours, and 12 hours -3d before administration, 0 hours, 1 hour, 2 hours, 4 hours, 8 hours, and 12 hours - 2d before administration, 0 hours, 1 hour, 2 hours, 4 hours, 8 hours, and 12 hours -1d before administration, 1d immediately before administration (0h), and 15 minutes, 30 minutes, 1 hour, 2 hours, 4 hours, 8 hours, 12 hours, and 24 hours (2d) after start of administration
- Evaluation variables: AUC_{inf}, AUCₗₐₛₜ, Cₘₐₓ, Tₘₐₓ, t_{1/2}
- The half-life of the drug calculated based on estimates made from non-clinical data was estimated to be approximately 1 hour.

### [Data analysis]

### • Analysis of safety and tolerability

Adverse events were described by dose group and treatment group based on severity and causal relationship with the stsudy drug, and the frequency and percentage of the number of test subjects with adverse events were compared and evaluated. Vital signs, electrocardiogram test, clinical laboratory tests, physical examination, monitoring of adverse events such as subjective and objective symptoms, continuous ECG and SpO2 monitoring, and the like were comprehensively reviewed, and statistical analysis was performed if necessary only on items determined to be clinically significant.

### • Pharmacokinetic evaluation

Pharmacokinetic variables were obtained by a non-compartmental analysis method for subjects who completed this clinical trial using appropriate and validated pharmacokinetic software (for example, Phoenix WinNonlin^{®} (Version 8.0 or higher, Pharsight, CA, USA)), and descriptive statistics (mean, standard deviation, and the like) were obtained for each dose group and treatment group.

### <Example 4> Examination of efficacy of TDCA as therapeutic agent for patients with coronavirus disease 2019 (COVID-19) - Phase 2 clinical trial

### <4-1> Selection of subject for phase 2 clinical trial

The subjects of the clinical trial were those who met all the criteria in [Table 4] among people infected with coronavirus disease 2019 (COVID-19) in Romania, Europe.

**[Table 4]**

| Order | Patient selection criteria |
|---|---|
| 1 | Male or female over 18 years of age but under 80 years of age |
| 2 | A case where SARS-CoV-2 infection is first confirmed by laboratory PCR test within 144 hours prior to randomization |
| 3 | A case where radiographic images (chest x-ray, CT scan) and three of the following clinical symptoms occur (new cough, fever, fatigue, sputum (all day), tachypnea, difficulty in breathing, and pleuritic chest pain) and a case where CRP > 10mg/L or more |
| 4 | A case where the white blood cell count in the patient's blood > 4.0 x 10⁹/L and lymphocyte count > 0.7 x 10⁹/L |
| 5 | A case where indoor SpO₂ ≤ 94% or Pₐ₀₂/F_{I02} ratio < 300 mmHg during the screening stage |
| 6 | Those who are determined to be appropriate to participate in a clinical trial and gives written consent to participate in the clinical trial of his or her own free will |
| 7 | For women of childbearing age, those who agree to continue using medically acceptable effective contraception until 90 days after the last administration in the clinical trial |

However, subjects who met the items shown in [Table 5] below were excluded.

**[Table 5]**

| Order | Patient exclusion criteria |
|---|---|
| 1 | A case where ALT (Alanine Transaminase) or AST (Aspartate Transaminase) > 5xULN |
| 2 | A case where eGFR < 30 ml/min and hemodialysis or hemofiltration is required because of decreased renal function |
| 3 | Pregnant or lactating women |
| 4 | A case where there are signs of multiple organ failure |
| 5 | A case where steroid is administered 72 hours before participation in the clinical trial |
| 6 | A case where there has been participation in a clinical trial related to COVID-19 |
| 7 | A case where the doctor judges that participating in the clinical trial is not the best option for the patient or that the clinical protocol cannot be safely performed |

During the clinical trial, subjects who met the criteria in [Table 6] dropped out.

**[Table 6]**

| Order | Patient dropout criteria |
|---|---|
| 1 | A case where serious side effects occur according to the clinical investigator's judgment, including lab abnormalities |
| 2 | A case where a patient does not properly comply with the clinical trial |
| 3 | A case where serious deviations from the protocol are discovered |
| 4 | A case where treatment with prohibited drugs (steroids) is required |
| 5 | A case where a patient gets pregnant midway through the clinical trial |
| 6 | A case where the principal investigator decides to discontinue the clinical trial on a patient for any reason |

### <4-2> Progress and management of clinical trial

In order to evaluate the efficacy of the therapeutic agent in the recruited patients for clinical trial, a randomized, double-blind, standard treatment-based placebo-controlled trial group was assigned, and the clinical trial was conducted. As a therapeutic agent, NuSepin, a therapeutic agent containing TDCA as an active ingredient, was used.

### [Progress stages]

### Patient screening (Day -5 to 0)

Patients who wanted to participate in the clinical trial signed the patient information sheet and informed consent form and then underwent a screening test. A screening number was assigned to each patient, and demographic information, patient medical history, past and current medication history, current medical history, vital signs (blood pressure, heart rate, body temperature, respiratory rate, level of consciousness), physical examination, electrocardiogram, laboratory tests, COVID-19 PCR test, arterial blood gas analysis, and chest x-ray or CT scan to evaluate pulmonary infiltration were performed. For women of childbearing age, a serum pregnancy test was performed.

### Setting of patient criteria (Day 1)

After monitoring of vital signs (blood pressure, heart rate, body temperature, respiratory rate, level of consciousness), transcutaneous oxygen saturation (SpO₂), physical examination, and evaluation of clinical trial inclusion/exclusion criteria were performed. The clinical status of the patient was evaluated using a 6-level scale.

Patients were randomly assigned to either a NuSepin 0.1 or 0.2 mg/kg group or a placebo group in which standard treatment using an antiviral drug was combined. Thereafter, the drug was administered intravenously two times a day from day 1 to day 14 or until the day of discharge. During the administration period, adverse drug events (AEs) and all details of the drugs administered to the patient were collected, recorded, and managed.

### Progress of clinical evaluation (Day 2 to Day 14)

For 2 to 14 days from the test date, vital signs (blood pressure, heart rate, body temperature, respiratory rate, level of consciousness), SpO₂, and physical examination were regularly monitored, the patient's clinical status was evaluated, and laboratory tests (serum chemistry analysis, blood tests, urinalysis, liver/renal function tests, inflammatory parameters), 12-lead ECG, adverse drug events, and changes in drug administered to the patient were collected and recorded. Depending on the judgment of the researcher and clinician, the participant's early discharge was decided, and those eligible for early discharge underwent follow-up management and final completion test process to collect research data.

### Follow-up management

After 14 days of the administration experiment, the collection of vital sign monitoring (blood pressure, heart rate, body temperature, respiratory rate, level of consciousness), SpO₂ measurement, 6-scale clinical status evaluation, physical examination, 12-lead ECG, urine pregnancy test (women of childbearing age), laboratory analysis (serum chemistry, urinalysis, liver/renal function tests, inflammatory marker tests), adverse drug events, and changes in drug during administration period was completed after the administration of the study drug was completed.

### Completion of clinical trial

Finally, on day 28, a clinical follow-up visit, physical examination, SpO₂ measurement, and laboratory analysis (serum chemistry, blood tests, urinalysis, liver/renal function tests, and inflammatory marker tests) were performed to collect information on adverse drug events, changes in drug concurrently used, and survival status.

### [Clinical evaluation indicators]

### 1. NEWS2 (National Early Warning Score 2)

The NEWS2 clinical index is a clinical evaluation index that is scored based on the measurement data such as whether an oxygen respirator is needed, body temperature, blood pressure, heart rate, body temperature, respiratory rate, level of consciousness, and oxygen saturation according to the criteria in [Table 7] and summed up to evaluate the degree of the patient's disease and to evaluate/determine the treatment stage. As a clinical evaluation index, NEWS2 was evaluated and recorded on days 1 to 15 and the end date. When the NEWS2 index was 5 or more, the patient was classified as a moderate or higher risk group, and subjected to subgroup analysis and stratification analysis as a patient in the high risk group. In addition, the time taken to maintain complete remission having a NEWS2 index of 0 for 24 hours was recorded and analyzed as NEWS2-based duration of treatment.

**[Table 7]**

| physiological parameter | NEWS2 Score | | | | | | |
|---|---|---|---|---|---|---|---|
| | 3 | 2 | 1 | 0 | 1 | 2 | 3 |
| respiration rate (per minute) | ≤8 | | 9-11 | 12-20 | | 21-24 | ≥25 |
| SpO₂ Scale 1(%) | ≤91 | 92-93 | 94-95 | ≥96 | | | |
| SpO₂ Scale 2 (%) | ≤83 | 84-85 | 86-87 | 88-92 ≥93 on air | 93-94 on oxygen | 95-96 on oxygen | ≥97 on oxygen |
| Air or oxygen? | | oxygen | | Air | | | |
| Systolic blood pressure (mmHg) | ≤90 | 91-100 | 101-110 | 111-219 | | | ≥220 |
| Pulse(per minute) | ≤40 | | 41-50 | 51-90 | 91-110 | 111-130 | ≥131 |
| Consciousness | | | | Alert | | | CVPU |
| Temperature (°C) | ≤35.0 | | 35.1-36.0 | 36.1-38.0 | 38.1-39.0 | ≥39.1 | |

### 2. OS (Ordinal scale)

OS is a clinical indicator that is divided into six scales and scored based on the criteria in [Table 8] according to the depth of oxygen breathing therapy, and lower scores mean that the patient's condition is better. As a clinical evaluation indicator for patients with COVID-19 pneumonia, OS was evaluated and recorded on days 1 to 15 and the end date.

**[Table 8]**

| Score | Criteria |
|---|---|
| 1 | Complete Clinical Remission |
| 2 | Hospitalization, not requiring supplemental oxygen |
| 3 | Hospitalization, requiring supplemental oxygen (such as Low Flow Nasal Canula or facial mask) |
| 4 | ICU/Hospitalization, requiring Non-invasive ventilation/High Flow Nasal Canula therapy |
| 5 | ICU, requiring Extracorporeal Membrane Oxygenation and/or invasive mechanical ventilation |
| 6 | Death |

### 3. Oxygen respiration treatment end date

As an indicator of respiratory function recovery in patients with COVID-19 pneumonia, the final end of oxygen respiration treatment was observed/recorded on days 1 to 15 and the end date.

### 4. CRP, IL-6, IL-8, TNF-α

CRP as an acute inflammatory factor and levels of IL-6, IL-8, and TNF-α as inflammatory cytokines in the blood were measured/recorded on days 1 to 15 and the end date in patients with COVID-19 pneumonia.

### Experimental Example 1> Examination of ability of TDCA to inhibit inflammation-related factor in in vitro mBMDM model

In order to examine whether TDCA can inhibit cytokine release syndrome caused by inflammatory signals, an experiment was conducted using an *in vitro* mBMDM model.

### <1-1> Examination of ability of TDCA to inhibit inflammatory cytokines IL-1β and TNF-α in inflammation priming signal

mBMDM were separated/extracted by the same method as that described in Example <1-2>, and the cells undergone inflammatory stimulation by LPS or the like were treated with TDCA to examine the inflammatory signal inhibiting effect of TDCA *in vitro.*

Specifically, in order to examine the effect of regulating the concentration of intracellular cAMP, which inhibits inflammatory factor-related gene transcription through the NF-κB pathway, a control group, an experimental group treated with 100 ng/ml LPS for 30 minutes, and an experimental group treated with 100 ng/ml LPS and 400 ng/ml TDCA simultaneously for 30 minutes were prepared in mBMDM cells, and the cAMP concentration in cells of each experimental group was measured by chemiluminescent analysis.

As a result, as illustrated in FIG. 1A, it has been confirmed that cAMP, which has been decreased by inflammatory signals caused by LPS, is recovered by TDCA.

Subsequently, in order to examine the efficacy of TDCA in inhibiting transcription of inflammation-related factors in an inflammatory state, the transcriptional expression of inflammation-related factors, ASC, NLRP3, Caspase1, and IL-1β, was measured through qRT-PCR experiments in the experimental group in which mBMDM were treated with 10 ng/ml LPS and 300 µM BzATP for 1 hour and the experimental group in which 10 ng/ml LPS, 300 µM BzATP, and 400 ng/ml TDCA were simultaneously treated for 1 hour, and the rate of decrease in expression by TDCA compared to that in the inflammatory state was analyzed.

As a result, as illustrated in FIG. 1B, it has been confirmed that the intracellular transcriptional expression of ASC, NLRP3, Caspase1, and IL-1β are all decreased by the treatment with TDCA in an inflammatory state caused by LPS+BzATP.

In order to examine the effect of TDCA on the expression of TNF-α, an inflammatory cytokine, TNF-α was measured in experimental groups in which mBMDM were treated with 10 ng/ml LPS for 3 hours and then with 0, 25, 100, and 400 ng/ml of TDCA for 1 hour, respectively, by ELISA and compared and analyzed.

As a result, as illustrated in FIG. 1C, it has been confirmed that TNF-α produced by LPS is decreased by the treatment with TDCA.

As illustrated in the schematic diagram of FIG. 9, through the above results, it has been confirmed that cAMP increased by TDCA inhibits NF-κB to reduce the expression of inflammatory factors such as ASC, NLRP3, and Caspase1, and as a result, TDCA has an inflammatory signal inhibiting effect to inhibit the expression of TNF-α, an inflammatory cytokine.

### <1-2> Examination of ability of TDCA to inhibit inflammatory cytokine IL-1β in inflammation activation signal

mBMDM were separated/extracted by the same method as that described in Example <1-3>, and the cells underwent inflammatory stimulation to examine changes in the expression and interaction of GPCR19-P2X7 by TDCA and the inflammatory cytokine IL-1β inhibiting efficacy of TDCA.

Specifically, in order to observe changes in the expression of and interaction between GPCR19 and P2X7 by TDCA, the expression and pattern of GPCR19 (green fluorescence signal) and P2X7 (red fluorescence signal) in mBMDM in a steady state, mBMDM simultaneously injected with 10 ng/ml LPS and 300 µM BzATP, and mBMDM treated with 400 ng/ml TDCA in a 10 ng/ml LPS + 300 µM BzATP stimulation state were examined through immunofluorescence staining and confocal microscopy.

As a result, as illustrated in FIG. 2A, it has been confirmed that the expression of GPCR19 decreases and, at the same time, the expression of P2X7 increases when inflammatory signals are activated and the decrease in GPCR19 and the increase in P2X7 are suppressed if treatment with TDCA is performed at this time. In addition, it has been confirmed that the co-localization fluorescence signal (yellow fluorescence signal) of GPCR19-P2X7 decreases when the inflammatory signal is activated and increases again by the treatment with TDCA.

In order to examine the inflammatory cytokine IL-1β inhibiting efficacy of TDCA, mBMDM in which inflammation was activated with LPS+ATP or LPS+BzATP were treated with 0, 25, 100, or 400 ng/ml of TDCA, and then the IL-1β concentration was measured by ELISA.

As a result, as illustrated in FIG. 2B, it has been confirmed that the concentration of IL-1β produced by inflammation activation decreases in proportion to the amount of TDCA used for treatment.

As illustrated in the schematic diagram of FIG. 9, through the above results, it can be seen that the expression of GPCR19 and P2X7R is regulated and the interaction between the two proteins increases by TDCA after inflammation is activated, the activation of NLRP3 inflammasome is inhibited by this signaling, and the production of inflammatory cytokine IL-1β is inhibited.

Through the results of <Experimental Example 1>, it can be seen that TDCA inhibits the excessive inflammatory response of immune cells and inhibits the production of TNF-α and IL-1β by the mechanism illustrated in the schematic diagram of FIG. 9. This suggests that TDCA can prevent and treat cytokine storm caused by SARS-CoV-2 infection.

### <Experimental Example 2> Examination of therapeutic effect of TDCA in in vivo systemic inflammation-induced animal model

Since patients with COVID-19 pneumonia are at increased risk of organ failure or death due to cytokine storm caused by systemic inflammation, an experiment was conducted to examine that TDCA can inhibit systemic inflammatory responses *in vivo.*

Specifically, when TDCA was administered three times at 12-hour intervals at 2 mg/kg, 1 mg/kg, and 1 mg/kg, respectively, to a large animal model in which systemic inflammation was induced by intravenous administration of LPS to dogs by the same method as that described in <Example 2>, the therapeutic effect to decrease the death rate and organ failure indicators and reduce cytokine storm in the animal experimental groups administered with TDCA was confirmed.

As a result, as illustrated in FIG. 3B, in a systemic inflammation-induced animal experiment, half of the animals have died at 48 hours, the end of the experiment, in the placebo group while all animals administered with TDCA have survived until 48 hours, and this shows the therapeutic effect of TDCA to improve the survival rate. As illustrated in FIG. 3C, when blood pressure, an indicator of cardiovascular failure, is observed, hypotensive shock occurs in the placebo animal group but hypotensive shock tends to be inhibited in the animal group administered with TDCA. As illustrated in FIGS. 3D and 3E, the levels of BUN and creatinine in the blood, which are indicators of renal failure, increase in the placebo group, but increases in the levels of BUN and creatine are suppressed in the group administered with TDCA. As illustrated in FIGS. 3F and 3G, IL-1β and IL-8, which are the causes of cytokine storm, are caused by systemic inflammation caused by LPS, and at this time, animals administered with TDCA show a therapeutic pattern in which cytokines are further inhibited than those in the placebo group.

The above results suggest the therapeutic effect of TDCA in reducing death rate, risk of organ failure, and cytokine storm *in vivo* where systemic inflammation is induced, and as a result, this suggests that death, organ failure, and tissue damage due to systemic inflammation, which are risk factors for patients with COVID-19 pneumonia, can be prevented/treated with TDCA.

### <Experimental Example 3> Examination of safety of TDCA in human body

In order to examine the drug safety/tolerability and pharmacokinetics (PK) of TDCA, a clinical trial was conducted by the same method as that described in <Example 3>.

As a result, it has been confirmed that three of the clinical trial subjects have side effects, one has mild IMD (immunomodulatory drugs)-related side effects, and all of the side effects are not severe and recover without treatment. It has also been confirmed that PK properties are exhibited in a concentration dependent manner.

Through the above results, it can be seen that TDCA exhibits excellent safety in the human body and tolerability.

### <Experimental Example 4> Examination of clinical therapeutic efficacy of TDCA for patient with COVID-19 pneumonia

In order to examine whether TDCA has therapeutic efficacy for actual patients with COVID-19 pneumonia, a clinical trial of NuSepin, an injectable study drug containing TDCA as an active ingredient, was conducted, and the clinical evaluation was monitored and the timing of treatment was checked to evaluate the efficacy.

Specifically, clinical patients were recruited and experiments were conducted by the same method as that described in <Example 4> to collect study drug efficacy evaluation data from 20 people in the placebo group and 22 people in the NuSepin 0.2 mg/kg intravenous administration group. The basic demographic indicators of clinical patients were as shown in [Table 9], and the distribution of disease severity was as shown in [Table 10] .

**[Table 9]**

| | | **NuSepin** (n=22) | **Placebo** (n=20) |
|---|---|---|---|
| **Gender** | | | |
| | Female | 10 (45%) | 10 (50%) |
| | Male | 12 (55%) | 10 (50%) |

| **Age, years** | | | |
|---|---|---|---|
| | Median (IQR) | 59 (54-68) | 63 (56-69) |
| | 18-60 years | 13 (59%) | 9 (45%) |
| | >60 years | 9 (41%) | 11 (55%) |

| **Race** | | | |
|---|---|---|---|
| | Caucasian | 22 (100%) | 20 (100%) |

| **Comorbidities** | | | |
|---|---|---|---|
| | Obesity | 6 (27%) | 4 (20%) |
| | Type 2 diabetes | 7 (32%) | 3 (15%) |
| | Hypertension | 12 (55%) | 14 (70%) |
| | Cardiac disorders | 3 (14%) | 3 (15%) |

| **Lab.** | | | |
|---|---|---|---|
| | C-reactive protein, mg/L | 77.1 (50.8) | 84.1 (62.7) |
| | TNFα, pg/ml | 10.3 (7.2) | 11.4 (3.2) |
| | IL-1β, pg/ml | 4.6 (5.4) | 3.5 (2.7) |
| | IL-6, pg/ml | 19.0 (18.4) | 51.0 (138.2) |
| | IL-8, pg/ml | 22.1 (18.7) | 22.0 (14.1) |
| | *N (%) or mean (SD) | | |

**[Table 10]**

| | | **NuSepin** (n=22) | **Placebo** (n=20) |
|---|---|---|---|
| **Disease severity** | | | |
| | Mild(NEWS2 < 5) | 4 (18%) | 7 (35%) |
| | Moderate (NEWS2 = 5 or 6) | 3 (14%) | 4 (20%) |
| | Severe (NEWS2 ≥ 7) | 15 (68%) | 9 (45%) |
| **Fever (temperature >37.3°C)** | | 8 (36%) | 3 (15%) |
| **Respiratory rate >24 breaths/min** | | 0 (0%) | 0 (0%) |
| **Pulse ≥125 beats/min** | | 0 (0%) | 0 (0%) |

| **Respiratory support** | | | |
|---|---|---|---|
| All patients requiring supplemental oxygen, N | | 21 | 19 |
| LFNC or facial mask | | 15 (71°) | 17 (89%) |
| Noninvasive ventilation orhigh-flow nasal cannula | | 6 (29%) | 2 (11%) |
| Moderate (NEWS2 5 and 6), N | | 3 | 4 |
| LFNC or facial mask | | 3 (100%) | 3 (75%) |
| Noninvasive ventilation orhigh-flow nasal cannula | | 0 (0%) | 1 (25%) |
| Severe (NEWS2 ≥ 7), N | | 15 | 9 |
| LFNC or facial mask | | 9 (60%) | 8 (89%) |
| Noninvasive ventilation orhigh-flow nasal cannula | | 6 (40%) | 1 (11%) |
| *N (%) | | | |

As illustrated in FIG. 4, the therapeutic effect was examined by calculating the recovery rate ratio using the Cox proportional hazard model from the duration of treatment based on NEWS2 clinical indicators (days required to maintain a NEWS2 score of 0 for 24 hours) for patients administered with NuSepin compared to that in the placebo group by reflecting underlying factors such as age, gender, underlying disease, severity, and drugs concurrently used. Specifically, it has been confirmed that there is a significant difference in the therapeutic effect by NuSepin when the use of antiviral drugs and patients in the moderate or higher risk group having a NEWS2 score of 5 or more are variables. To explain in detail, when antiviral drug variable is reflected, there is a significant recovery increase effect as the recovery rate ratio of patients administered with NuSepin to the placebo group is 3.7 [95% C.I. 1.4 to 10.2]] and p = 0.01. When analysis is performed by reflecting the symptomatic variable of NEWS2 ≥ 5, there is a significant recovery increase effect as the recovery rate ratio of the NuSepin administration group to the placebo group is 2.7 [95% C.I. 1.2 to 6.4] and p = 0.02. It has been confirmed that there is a significant recovery increase effect as the recovery rate ratio of patients administered with NuSepin to the placebo group is 3.4 [95% C.I. 1.5 to 7.4] ] and p = 0.003 when the concurrent use of antiviral drugs and the critical illness (NEWS2 ≥ 5) variable are simultaneously reflected in the analysis. The Kaplan-Meier survival curve comparison diagram illustrated in FIG. 5 is the result of comparing the cumulative pattern of NEWS2-based duration of treatment in the group of moderate to severe patients having NEWS2 of 5 or more, and shows that there is a therapeutic effect to shorten the duration of treatment in the NuSepin administration group compared to the placebo control group. Specifically, when comparison is performed on moderate to severe ITT (intend to treat) patients, it has been confirmed that the duration of treatment is mathematically shortened by 3.5 days as the median value of NEWS2-based duration of treatment is 12.5 [95% C.I. 8.0 to 25.0] days in the placebo group but is 9.0 [95% C.I. 7.0 to 12.0] days in the NuSepin administration group, and the recovery rate ratio is improved to 1.9 [95% C.I. 0.9 to 4.3]. When comparison is performed on PP (Per protocol) patients, it has been confirmed that the duration of treatment is shortened by 3.5 days as the NEWS2-based duration of treatment is 12.5 [95% C.I. 8.0 to 25.0] days in the placebo group but is 9.0 [95% C.I. 7.0 to 11.0] days in the NuSepin administration group, and there is a statistically significant difference as the statistical significance calculated by the Log-Rank test is p = 0.016. In addition, it has been confirmed that there is a significant recovery increase effect as the recovery rate ratio calculated using the Cox proportional hazard model is 2.7 [95% C.I. 1.2 to 6.4] and p=0.02 at this time.

As illustrated in FIG. 6, when the recovery rate ratio is calculated using the time taken until the end date of oxygen respiration treatment as an evaluation indicator and the severity (NEWS2≥5) as an adjustment variable, it has been confirmed that there is a therapeutic effect to end oxygen respiration treatment earlier in the NuSepin treatment group than in the placebo group in moderate to severe patients. Specifically, it has been confirmed that NuSepin administration is significant as oxygen respiration treatment ends in 9 days [95% C.I. 7.9 to 10.1] in the placebo group but in 8 days [95% C.I. 7.4 to 8.6] in the administration group, reducing the duration of oxygen respiration treatment by 1 day, and as the recovery rate ratio calculated using the Cox proportional hazard model is 2.2 [95% C.I. 1.0 to 4.6] and the statistical significance is p = 0.04.

As illustrated in FIG. 7, in order to examine the effect of improving respiratory-based OS clinical indicators, the rate of decrease in OS score on day 9 of the clinical trial compared to that on the clinical trial start date was compared and confirmed, and as a result, it has been confirmed that there is an improvement effect as the NuSepin administration group has a higher rate of decrease than the placebo group. Specifically, in all patients, the average rate of decrease in OS score was 30.9% in the placebo group but was 43.9% in the NuSepin administration group. In moderate to severe patients having NEWS2 of 5 or more, the OS score decreased by 24.3% in the placebo group but by 41.7% in the NuSepin administration group, and the statistical significance calculated by the Wilcoxon rank sum test was p = 0.028, confirming that the difference in drug efficacy between the placebo group and the NuSepin administration group is significant.

As illustrated in FIG. 8, it has been confirmed that there is a therapeutic effect that the proportion of patients whose CRP and cytokine levels return to normal is higher in the NuSepin administration group than in the placebo group in all patients. Specifically, on day 4 of the clinical trial, CRP reached the normal level (less than 10 mg/ml) in 200 of the placebo group but in 52% of the administration group. On the last day of the clinical trial, the TNF-α level in the blood reached the normal level (less than 6 pg/ml) in 11% of the placebo group but in 35% of the administration group. On the end date, the mean TNF-α level (±SEM) was 24.8 (±12.3) in the placebo group but was as low as 7.4 (±0.7) in the NuSepin group, and the statistical significance calculated by the Wilcoxon rank sum test was p = 0.007, confirming that the difference in drug efficacy between the placebo group and the NuSepin administration group is significant. On the last date of the clinical trial, the IL-8 level in the blood reached the normal level (less than 10 mg/ml) in 33% of the placebo group but in 55% of the administration group. The IL-8 level at the end of the clinical trial was not significantly lower than that on the start date in the placebo group, but the IL-8 level decreased from 22.1 (±4.0) at the start of the clinical trial to 12.8 (±2.2) on the end date of the clinical trial in the NuSepin treatment group, and the statistical significance calculated by the Wilcoxon rank sum test was p < 0.05, confirming that this is a significant decrease due to the therapeutic agent. At the end of the clinical trial, IL-6 reached the normal level (less than 3.3 pg/ml) in 33% of the placebo group but in 60% of the administration group.

Through the results of phase 2 clinical trial, it has been confirmed that NuSepin has drug efficacy effectiveness to reduce the duration of treatment for patients with COVID-19 pneumonia and inhibit systemic inflammation.

In addition, as shown in Table 11, there were no cases of serious side effects caused by the administration of NuSepin 0.1 mg/kg and 0.2 mg/kg during the clinical trial, and the number of side effects was small and was not different from that in the placebo group. The side effects occurred were very mild and were confirmed to be temporary symptoms that all recovered during the clinical trial. Through this, the safety and excellent tolerability of TDCA, the therapeutic agent in this experiment, have been confirmed.

**[Table 11]**

| | **NuSepin 0.1 mg/kg (n=22)** | **NuSepin 0.2 mg/kg (n=22)** | **Placebo (n=20)** |
|---|---|---|---|
| **Patients with ≥1 adverse event, No. (%)** | 6 (27) | 6 (27) | 4 (20) |

| Patients with severe adverse event | | | |
|---|---|---|---|
| Any severe adverse event | 0 (0) | 1 (5) | 0 (0) |
| Study drug related severe adverse event | 0 (0) | 0 (0) | 0 (0) |

| Relationship | | | |
|---|---|---|---|
| Possibly | 5 (23) | 3 (13) | 2 (10) |
| Unassesible | 1 (5) | 3 (13) | 2 (10) |
| Fatal event | 0 (0) | 0 (0) | 0 (0) |

| **Patients with adverse events of specific organs, No. (%)** | | | |
|---|---|---|---|
| - **Blood system** | | | |
| Anemia | 1 (5) | 1 (5) | 0 (0) |
| Leukocytosis | 1 (5) | 0 (0) | 0 (0) |
| Neutrophilia | 1 (5) | 0 (0) | 0 (0) |

| - **Cardiovascular system** | | | |
|---|---|---|---|
| Tachycardia | 1 (5) | 0 (0) | 3 (15) |
| Hypotension | 1 (5) | 1 (5) | 0 (0) |

| - **Gastrointestinal tract** | | | |
|---|---|---|---|
| Diarrhea | 0 (0) | 1 (5) | 0 (0) |

| - **Hepatobiliary system** | | | |
|---|---|---|---|
| ALT increased | 1 (5) | 2 (9) | 2 (10) |
| AST increased | 2 (9) | 2 (9) | 0 (0) |
| GGT increased | 2 (9) | 2 (9) | 0 (0) |

| - **Metabolism** | | | |
|---|---|---|---|
| Hypokalemia | 0 (0) | 1 (5) | 0 (0) |
| - **Nervous system** | | | |
| Headache | 0 (0) | 2 (9) | 2 (10) |

| - **Thorax** | | | |
|---|---|---|---|
| Dyspnea | 0 (0) | 1 (5)* | 0 (0) |

| - **Skin and subcutaneous tissue** | | | |
|---|---|---|---|
| Exanthema | 1 (5) | 0 (0) | 0 (0) |
| * Severe adverse event, not related to NuSepin | | | |

As a result, the results of <Experimental Example 4> suggest that TDCA inhibits excessive inflammatory response of immune cells and exhibits an effective therapeutic effect on patients with COVID-19 pneumonia by the mechanism illustrated in the schematic diagram of FIG. 9.

### [Industrial Applicability]

Taurodeoxycholic acid (TDCA) or a pharmaceutically acceptable salt thereof according to the present invention treats clinical symptoms caused by novel severe acute respiratory syndrome coronavirus 2 (SARS-CoV-2) infection, exhibits an early full recovery effect through inflammation alleviation and inflammatory cytokine inhibitory efficacy, and can be thus used as an active ingredient in a composition for prevention or treatment of lower respiratory tract infectious diseases, including coronavirus disease 2019.

## Claims

1. A pharmaceutical composition for prevention or treatment of lower respiratory tract infectious diseases, comprising taurodeoxycholic acid or a pharmaceutically acceptable salt thereof as an active ingredient.

2. The pharmaceutical composition according to claim 1, wherein the taurodeoxycholic acid is a compound represented by the following [Chemical Formula 2]:

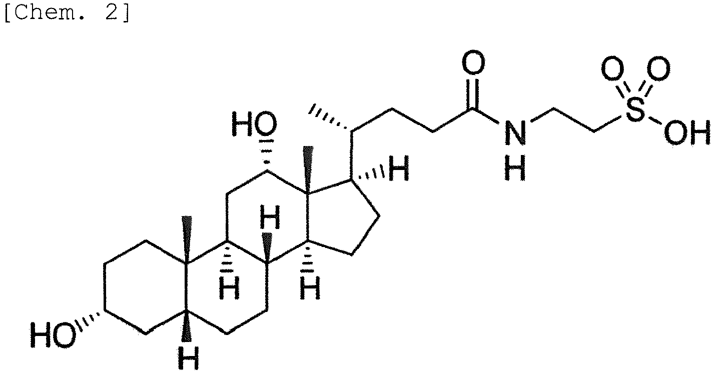

3. The pharmaceutical composition according to claim 1, wherein the salt is a sodium salt.

4. The pharmaceutical composition according to claim 1, wherein the taurodeoxycholic acid or a pharmaceutically acceptable salt thereof inhibits production of inflammatory cytokines selected from the group consisting of TNF-α, IL-1β, IL-8, IL-6, and any combination thereof.

5. The pharmaceutical composition according to claim 1, wherein the taurodeoxycholic acid or a pharmaceutically acceptable salt thereof is a GPCR19 agonist that inhibits activation of NF-κB by increasing cAMP and inhibits activation of NLRP3 inflammasome by inhibiting a function of P2X7.

6. The pharmaceutical composition according to claim 1, wherein the lower respiratory tract infectious disease is a disease having one or more symptoms selected from the group consisting of fever, cough, phlegm, body aches, sore throat, diarrhea, conjunctivitis, headache, skin rash, difficulty in breathing, acute bronchitis, pneumonia, pulmonary suppuration, renal failure, renal dysfunction, septic shock, sepsis, and cytokine release syndrome caused by coronavirus infection.

7. The pharmaceutical composition according to claim 6, wherein the coronavirus is one or more selected from the group consisting of Middle East respiratory syndrome coronavirus (MERS-CoV), severe acute respiratory syndrome coronavirus (SARS-CoV), and novel severe acute respiratory syndrome coronavirus 2 (SARS-CoV-2).

8. A method for preventing lower respiratory tract infectious diseases, the method comprising administering taurodeoxycholic acid or a pharmaceutically acceptable salt thereof to an individual.

9. A method for treating lower respiratory tract infectious diseases, the method comprising administering taurodeoxycholic acid or a pharmaceutically acceptable salt thereof to an individual.

10. A use of taurodeoxycholic acid or a pharmaceutically acceptable salt thereof for preparing a pharmaceutical composition for prevention or treatment of lower respiratory tract infectious diseases.
